# EUROPEAN PATENT SPECIFICATION

(11) **EP 0 587 964 B1**
(45) Date of publication and mention of the grant of the patent: **18.02.1998**
(21) Application number: 92830490.6
(22) Date of filing: 16.09.1992
(51) Int. Cl.: A61B 3/11, G02C 13/00

(54) **Instrument for centering corrective lenses**
Zentriergerät für Augenlinsen
Instrument destiné au centrage de verres correcteurs

(43) Date of publication of application: 23.03.1994
(73) Proprietor: Minichiello, Angie, I-40026 Imola (Bologna) (IT)
(72) Inventor: Minichiello, Angie, I-40026 Imola (Bologna) (IT)
(74) Representative: Gardi, Giuliano

(56) References cited:
- DE-B- 1 197 247
- DE-U- 8 812 065
- FR-A- 2 384 481
- US-A- 3 410 637

## Description

The invention refers to an optical instrument for the centering of corrective lenses and to a method for centering corrective lenses for eyeglasses. For the correction of ametropia lenses are prescribed to the patient by the oculist and are then prepared by the optician who must apply the same on the frame. In order to center said lenses the optician, by means of a binocular unit (the interpupillometer) conventionally measures the interpupillary distance of the person and on the base of this he operates on the glass frame.

DE-A-1.197.247 discloses an instrument for measuring the semi-interpupillary distance from the middle of each eye to the mid-bridge of the nose. The alignment is achieved by an expert operator monocularly without error of parallax, since the patient fixes the image of his own pupil reflected from a mirror while the operator aligns the image of his own pupil with the patient's pupil.

US-A-3.410.637 discloses an instrument for measuring the interpupillary distance via two monocular semi-pupillary measures from the midline of the bridge of the nose.

The use of the interpupillary distance for centering corrective lenses for eyeglasses is an approximate and imprecise measure since it is not a precise measure of the inter-foveal distance between both eyes. Therefore a visual fatigue and a degradation of the retinal image is often generated due to induced prismatic effects, optical aberrations, coma, anisophoria, etc.

The problem solved by the present invention is to improve conventional systems for centering of corrective lenses so that the visual inconvenience outlined above are eliminated.

According to one aspect of the present invention, there is provided an instrument for the centering of corrective lenses for eyeglasses as defined in claim 1.

According to a further aspect of the present invention, there is provided a method for centering of corrective lenses for eyeglasses as defined in claim 9.

Owing to the invention, it is possible to center the optical axes of corrective lenses for eyeglasses by using the distance between the visual axes of the eyes of a patient, thereby substantially eliminating any defect induced by optical aberration.

The instrument according to the invention is based on the recognition that, in order to avoid induced prismatic effects and a degradation of the retinal image, it is necessary that the optical centres of the lenses were placed where come to pass the visual axes.

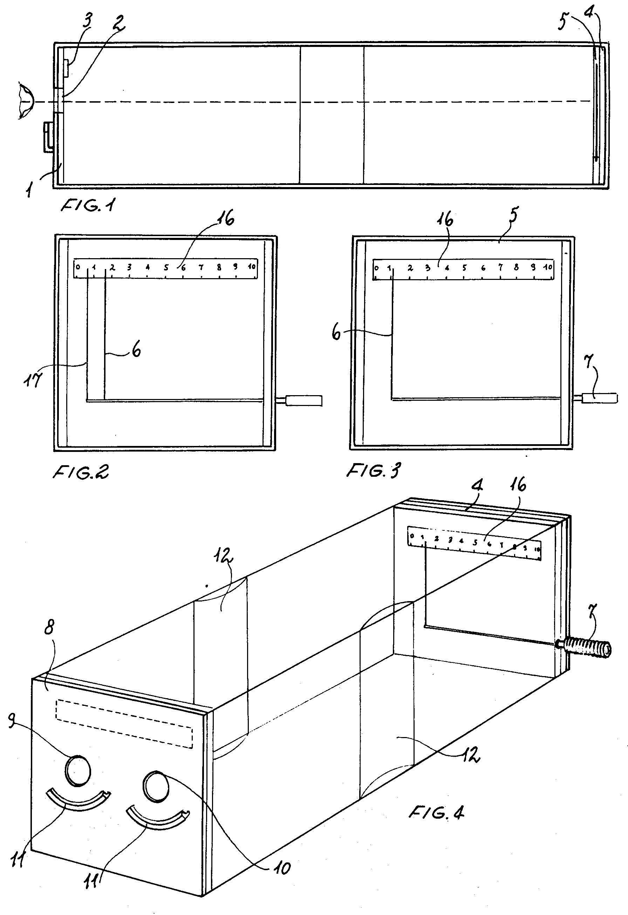

Therefore, the instrument is provided with two opposite mirrors to operate a total image reflection which permits to determine the distance between said visual axes of the examined person acting by two consecutive monocular bearings. The distance is computed by difference of the millimetric measures read in said bearings onto the reflex image on the mirror, as provided on a bottom wall of the instrument chamber, said distance being detected by means of a graduated scale for achieving in each bearing a measure via a micrometric translation regulator coupled to manual or electronic driving means. Substantially the invented instrument foresees a closed frame to form an inside parallelepiped reflexion chamber including on its front and back walls a pair of oppositely disposed parallel mirrors; a first mirror 1, co-operating with a pair of circular plate areas 2 with semireflecting effect to permit the bearings to be taken and having at its upper portion a millimetric scale 3, and a second mirror 4 on which is movable a mobile frame 5 supporting a bearing reference with mark rod 6 manually transferable by a micrometric regulator with a knob 7. On a frontal wall 8 of the instrument, where the circular plate areas 2 of the mirror 1 operating with semireflecting effect are located, observation holes 9 and 10 are provided and parabolic guides 11 for test corrective lenses are also provided. The reflexion chamber is illuminated by a pair of of bright diffuser 12 disposed in the middle of lateral walls of the casing. In use, the optician applies, as prescribed by the oculist, the test corrective lenses 13 and 14 on the parabolic guides 11 and switches on the light into the reflexion chamber. The person effects a first monocular sighting for the first eye through a respective observation hole 9 or 10. Owing to the mirror arrangement, the person will see on the bottom mirror 4, in specular position to the real disposition, the reflected images 16 of the millimetric scale 3 and, if said rod does not coincide with the visual axis of the eye under observation, he sees a set of images: the real rod 6 and a reflected image 17 thereof. The person will move the knob 7 of the micrometric regulator for movement of the frame 5 which bears the rod until the real image and the reflected image lie over each other and only the real image 6 can be seen: in this condition the rod is aligned with the visual axis of the first eye. The person reads then the measure of the refleted image on the reflected millimetric scale. A second monocular sighting is carried out analogously for the other eye and a second visual axis is identified. The millimeteric distance between the first and second visual axis is calculated by difference of the two measures. A preferred embodiment of the instrument is shown in drawings of sheets 1 and 2. In sheet 1, Fig. 1 is a longitudinal section of the instrument showing the operative system included into the reflection chamber. Fig. 2 is a front view of the bottom mirror 4 with the sighting mark with the rod 6 not in alignment with the visual axis of the person looking; Fig. 3 is a view as in Fig. 2 but with the rod brought in a centering position by knob 7. Fig. 4 is a perspective view of the casing of the instrument shown as with transparent walls to show the interior of the reflexion chamber.

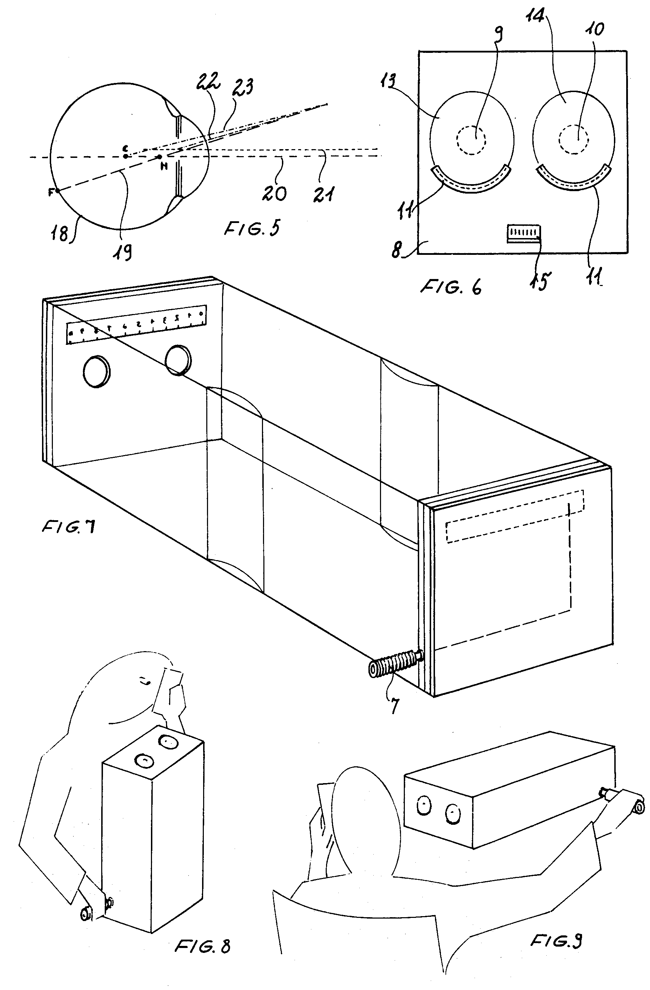

In sheet 2, Fig. 5 is a schematic view of the scientific principle to which the invented instrument is conformed. In Fig. 5, the retina 18 of the eyeball is indicated with F pointing at the fovea centralis, where the sight is sharper, point N indicating the intersection between the visual axis 19 and the optic axis 20. The pupillar axis 21, the line of sight 22 and the fixation axis 23 are also indicated, the fixation axis intersecting the optic axis in point C. Fig. 6 is a front view of the instrument showing test corrective lenses 13 and 14 in place on parabolic guides 11. Fig. 7 is a perspective view of the closed casing of the instrument as with transparent walls and seen from the back side. Fig. 8 is a view with a person during a monocular observation using an instrument with parallelepiped casing placed in elevation on a support plane. Fig. 9 is a view showing a person during a monocular observation using the instrument with a parallelepiped casing horizontally disposed. According to an embodiment not shown, movement of frame 5 to move the rod 6 in position on the bearing mark is achieved electronically.

## Claims

1. An instrument for centering of corrective lenses (13) for eyeglasses, the instrument having a casing with two opposed mirrors (1, 4) in the form of a parallelepiped reflexion chamber, comprising sighting means (1, 4, 6, 7) for detecting in sequence the position of both visual axes of an observer via two consecutive monocular sighting operations and measuring means (16) for measuring the distance between the detected visual axes.

2. An instrument according to claim 1, wherein said bearing means comprises opposite first (1) and second (4) mirrors disposed in parallel, the first mirror (1) having a pair of semi-reflecting circular plate areas (2) spaced apart from one another.

3. An instrument according to claim 2, wherein a movable frame (5) supporting a sighting mark rod (6) is positioned in front of the second mirror (4).

4. An instrument according to claim 2 and 3, wherein said sighting means further comprises a micrometric regulator mechanism with driving means (7) for moving the movable frame parallel to the second mirror (4).

5. An instrument according to claim 2, wherein observation holes (9, 10) are provided in the front wall (8) of the casing, the holes being positioned in alignment with the pair of semireflecting circular plate areas (2).

6. An instrument according to claim 2, wherein parabolic guide means (11) are arranged on the front wall (8) of the casing for guiding the correcting lenses (13) with respect to the observation holes (9, 10).

7. An instrument according to claim 2, wherein said measuring means includes a millimeter scale (3) positioned between the circular plate areas (2) and one edge of the first mirror.

8. An instrument according to claim 1, and further comprising means for illuminating the reflexion chamber, including a pair of diffusors (12) and a switch (15), wherein the diffusors (12) are disposed on the lateral walls of the casing.

9. A method for centering of corrective lenses for eyeglasses, wherein an observer sights monocularly with one eye and subsequently monocularly with the other eye to bring, in sequence and for each individual eye, a reflected image (17) of a mark rod (6) into alignment with the corresponding real mark rod so that the positions of the visual axes of both eyes are defined and the distance of one visual axis from the other visual axis is thus obtained.

## Patentansprüche

1. Instrument zum Zentrieren von Korrekturlinsen (13) für Brillen, mit einem zwei einander gegenüberliegende Spiegel (1, 4) aufweisenden Gehäuse in Form einer quaderförmigen Reflektionskammer, die Visiermittel (1, 4, 6, 7) zum Erfassen der Position beider visueller Achsen eines Beobachters nacheinander über zwei aufeinanderfolgende monokulare Visiervorgänge und Messmittel (16) zum Massen des Abstandes zwischen den erfassten visuellen Achsen aufweist.

2. Instrument nach Anspruch 1, dadurch gekennzeichnet, dass die Visiermittel gegenüberliegende erste (1) und zweite (4) Spiegel aufweist, die parallel zueinander angeordnet sind, wobei der erste Spiegel (1) ein paar semireflektierender kreisförmiger Scheibenflächen (2) aufweist, die im Abstand voneinander angeordnet sind.

3. Instrument nach Anspruch 2, dadurch gekennzeichnet, dass ein bewegbarer Rahmen, der eine Visiermarkierungsstange (6) trägt, vor dem zweiten Spiegel (4) angeordnet ist.

4. Instrument nach den Ansprüchen 2 und 3, dadurch gekennzeichnet, dass die Visiermittel ferner einen Micrometereinstellmechanismus mit Antriebsmitteln (7) zum Bewegen des bewegbaren Rahmens parallel zum zweiten Spiegel (4) aufweisen.

5. Instrument nach Anspruch 2, dadurch gekennzeichnet, dass Beobachtungsöffnungen (9, 10) in der Vorderwand (8) des Gehäuses vorgesehen sind, welche Öffnungen in Flucht mit dem Paar der semireflektierenden kreisförmigen Scheibenflächen (2) angeordnet sind.

6. Instrument nach Anspruch 2, dadurch gekennzeichnet, dass parabolförmige Führungsmittel (11) and der Vorderwand (8) des Gehäuses zum Führen der Korrekturlinsen (13) gegenüber den Beobachtungsöffnungen (9, 10) angeordnet sind.

7. Instrument nach Anspruch 2, dadurch gekennzeichnet, dass die Messmittel eine Millimeterskala (3) aufweisen, die zwischen den kreisförmigen Scheibenflächen (2) und einer Kante des ersten Spiegels angeordnet sind.

8. Instrument nach Anspruch 1, beinhaltend ferner Mittel zum Beleuchten der Reflektionskammer, die ein Paar Diffusoren (12) und einem Schalter (15) aufweisen, wobei die Diffusoren (12) an den Seitenwänden des Gehäuses angeordnet sind.

9. Verfahren zum Zentrieren von Korrekturlinsen für Brillen, dadurch gekennzeichnet, dass ein Beobachter monokular mit dem einen Auge und danach monokular mit dem anderen Auge visiert, um aufeinanderfolgend und für jedes Auge einzeln ein reflektiertes Abbild (17) einer Markierungsstange (6) in Flucht mit der betreffenden wirklichen Markierungsstange zu bringen, so dass die Positionen der visuellen Achsen beider Augen definiert sind und somit der Abstand der einen visuellen Achse von der anderen visuellen Achse erhalten wird.

## Revendications

1. Instrument pour le centrage de verres correcteurs (13) pour lunettes, l'instrument ayant un boîtier avec deux miroirs opposés (1, 4) sous la forme d'une chambre de réflexion parallélépipédique, comprenant des moyens de visée (1, 4, 6, 7) pour détecter successivement la position des deux axes visuels d'un observateur vis deux opérations de visée monoculaire consécutives, et des moyens de mesure (16) pour mesurer la distance entre les axes visuels détectés.

2. Instrument suivant la revendication 1, caractérisé en ce que lesdits moyens de visée comprennent un premier miroir (1) et un deuxième miroir (4) opposés et disposés en parallèle, le premier miroir (1) comportant deux zones de plaques circulaires semi-réfléchissantes (2) mutuellement espacées.

3. Instrument suivant la revendication 2, caractérisé en ce qu'un cadre-mobile (5) supportant une tige d'index de visée (6) est placé à l'avant du deuxième miroir (4).

4. Instrument suivant la revendication 2 ou 3, caractérisé en ce que lesdits moyens de visée comprennent en outre un mécanisme de réglage micrométrique pourvu de moyens d'entraînement (7) pour déplacer le cadre mobile parallèlement au deuxième miroir (4).

5. Instrument suivant la revendication 2, caractérisé en ce que des trous d'observation (9, 10) sont prévus dans la paroi avant (8) du boîtier, les trous étant placés en alignement avec les deux zones de plaques circulaires semi-réfléchissantes (2).

6. Instrument suivant la revendication 2, caractérisé en ce que des moyens de guidage paraboliques (11) sont prévus sur la paroi avant (8) du boîtier, pour guider les verres correcteurs (13) par rapport aux trous d'observations (9, 10).

7. Instrument suivant la revendication 2, caractérisé en ce que lesdits moyens de mesure comprennent une échelle millimétrique (3) placée entre les zones de plaques circulaires (2) et un bord du premier miroir.

8. Instrument suivant la revendication 1, caractérisé en ce qu'il comprend en outre des moyens d'éclairage de la chambre de réflexion, comportant deux diffuseurs (12) et un interrupteur (15), et en ce que les diffuseurs (12) sont disposés sur les parois latérales du boîtier.

9. Procédé de centrage de verres correcteurs pour lunettes, caractérisé en ce qu'un observateur regarde de façon monoculaire avec un oeil et ensuite de façon monoculaire avec l'autre oeil afin d'amener, en séquence et pour chaque oeil individuel, une image réfléchie (17) d'une tige d'index (6) en alignement avec la tige d'index réelle correspondante, de sorte que les positions des axes visuels des deux yeux sont définies et qu'on obtient ainsi la distance d'un axe visuel à l'autre axe visuel.
